# EUROPEAN PATENT SPECIFICATION

(11) **EP 0 648 271 B1**
(45) Date of publication and mention of the grant of the patent: **16.04.2003**
(21) Application number: 92918712.8
(22) Date of filing: 20.08.1992
(51) Int. Cl.: C12N 15/861, A61K 48/00, A61K 9/48

(54) **ADENOVIRUS MEDIATED TRANSFER OF GENES TO THE GASTROINTESTINAL TRACT**
ADENOVIRUS VERMITTELTER GENTRANSFER IN DEN GASTROINTESTINALTRAKT
TRANSFERT INDUIT PAR ADENOVIRUS DE GENES VERS LA VOIE GASTRO-INTESTINALE

(30) Priority: 20.08.1991 US 747371
(43) Date of publication of application: 19.04.1995
(73) Proprietor: THE GOVERNMENT OF THE UNITED STATES OF AMERICA as represented by the SECRETARY OF THE DEPARTMENT OF HEALTH AND HUMAN SERVICES, Bethesda, MD 20892-9902 (US)
(72) Inventor: CRYSTAL, Ronald, G., Potomac, MD 20854 (US)
(74) Representative: Armitage, Ian Michael
(86) International application number: US9207029
(87) International publication number: WO93003769

(56) References cited:
- US-A- 4 920 209
- US-A- 4 980 286
- Science, Volume 252, issued 19 April 1991, ROSENFELD et al., "Adenovirus-Mediated Transfer of a Recombinant Alpha1-Antitrypsin Gene to the Lung Epithelium in Vivo", pages 431-434, see the entire document.
- Biotechniques 6, issued 1989, BERKNER, "Development of Adenovirus Vectors for the Expression of Heterologous Genes", pages 616-629, see the entire document.
- FEBS Letters, Volume 267, Number 1, issued July 1990, GILARDI et al., "Expression of Human Alpha1-Antitrypsin Using a Recombinant Adenovirus Vector", pages 60-62, see the entire document.

## Description

### Field of the Invention

The present invention relates, in general, to use of adenovirus for transfer of genes to the gastrointestinal tract. In particular, the present invention relates to a use of recombinant, replication-deficient adenovirus in the manufacture of medicament or treatment of an animal by transfer of therapeutic genes to the gastrointestinal tract of the animal for the purpose of producing therapeutic proteins, locally or systemically.

### Background Information

The use of proteins as therapeutic agents is limited inter alia by the physiologic barrier of the gastrointestinal tract. The terms protein, polypeptide, peptide, or segment of amino acids are herein used interchangeably to define a polymer of amino acids linked through peptide bonds. Therapeutic proteins are defined herein as proteins advantageous to an individual. Proteins cannot be administered for therapeutic purposes by the oral or rectal routes because they will not generally reach the circulation in an intact form in concentrations needed for therapy (the proteins are degraded and/or not absorbed). Consequently, therapeutic proteins need to be administered systemically for example, by the intravenous, subcutaneous, intradermal or intramuscular routes.

This problem of the administration has been dramatically heightened by the development of recombinant DNA technology, where it is possible to produce many different therapeutic proteins, all of which have to be administered systemically. While this may not be a major problem for short term use, long term use (which is the typical use for most of the recombinant proteins) requires long term systemic administration with all of the attendant problems with access route (e.g., veins available, discomfort and cost).

It is known that recombinant adenoviruses can be used to produce human protein in vivo (examples include injection of recombinant adenovirus intravenously into the portal vein to the liver, and intratracheal to the lung (see Rosenfeld M et al. (1991) Science 252:431-434; Jaffe HA et al. (1991) Clin Res 39(2) 302A; Rosenfeld MA et al. (1991) Clin Res 39(2): 311A). However, all of these approaches are impractical to use for systemic administration of recombinant proteins because they require parenteral administration of the recombinant gene (i.e., intravenous, intraportal, intratracheal).

The present invention circumvents this by providing for administration of therapeutic proteins by enteral routes by using a recombinant, replication deficient adenovirus containing the coding sequences of the gene of the therapeutic protein to insert the gene into the lining cells of the gastrointestinal tract, and using that site to produce the protein and secrete it into the circulation where the therapeutic protein would be available for systemic use. As an alternative, the same approach can be used to secrete proteins into the gastrointestinal tract for local therapeutic use within the lumen of the gastrointestinal tract, or for use within the cells or extracellular matrix of the walls of the gastrointestinal tract.

Studies in the 1960's demonstrated that live adenovirus placed into enteric coated capsules (to avoid inactivation in the stomach) and administered to humans by the oral route resulted in systemic immunization against the adenovirus (Chanock RM et al. (1966) JAMA 195:151-158). This is now a standard immunization procedure against adenovirus for military recruits in the USA. The concept underlying this immunization strategy is that the adenovirus will leave the capsule as it dissolves in the lumen of the intestine, infect the intestinal epithelial cells, replicate in the epithelial cells and the resulting shed newly replicated virus presents itself to the immune system, resulting in systemic immunity against the adenovirus.

Previously, it has been demonstrated that the adenovirus can be modified so that it is replication deficient (i.e., will not direct the production of new virus after it infects its target cell) and so that it contains new genes (e.g., the coding sequences of human genes of therapeutic interest). The use of recombinant DNA inserts under the direct control of the early promoter (EP) of the Ela region of the adenovirus genome has been described by M. Perricaudet, et al. of the Pasteur Institute in European Patent Application No. 0185573, published June 25, 1986. Such a modified virus can be used to transfer the recombinant gene to target cells in vivo (for examples, see Rosenfeld M et al. (1991) Science 252:431-434; Berkner KL (1988) BioTechniques 6:616-629).

### SUMMARY OF THE INVENTION

It is a general object of this invention to provide for production of a protein in the cells of the gastrointestinal tract of an animal.

More specifically, this invention provides the use of a composition comprising a replication deficient adenovirus comprising a DNA segment encoding a biologically active protein and no virus other than said replication deficient adenovirus, for production of the protein in the gastrointestinal tract of an animal for a non-therapeutic and non-diagnostic purpose.

In another aspect, the invention provides the use of a replication deficient adenovirus comprising a DNA segment encoding a biologically active therapeutic protein in the manufacture of a medicament for treatment, by administration to the gastrointestinal tract of an animal for production of the protein in the gastrointestinal tract, of a disorder wherein the protein has a therapeutic effect, which medicament contains no virus other than said replication deficient adenovirus.

Depending on the specific sequences placed into the recombinant adenovirus, the protein would preferably be secreted for systemic therapy to the circulation, for local therapy to the lumen of the gastrointestinal tract, or both. The design of a replication deficient adenovirus.of the invention may preferably be to deliver the protein for use within the cells of the gastrointestinal tract or in the walls of the gastrointestinal tract.

Further objects and advantages of the present invention will be clear from the description that follows.

### BRIEF DESCRIPTION OF THE DRAWINGS

Figure 1. Recombinant adenovirus (Ad) vector. Top - wild type Ad5 genome showing the Ela, Elb [map units (mu) 1.3-11.2; 100 mu = 36 kb] and E3 (mu 7.6.6-86.0) regions.
Figure 2. Anatomy of the colon wall and the cultured epithelial cell model used to evaluate the polarity of secretion of α1-antitrypsin produced by T84 human colon carcinoma epithelial cells modified with the recombinant adenovirus Ad-α1AT (ATCC CCL 248).
   A. Cross-section of the colon wall showing the epithelial cells (14), the lumen of the colon (13), the apical surface (12) of the epithelium abutting the lumen, the basolateral surface (11) of the epithelium abutting the submucosa and thus the capillaries (19) and the muscle layer (20).
   B. Chamber for epithelial cell cultures showing the microporous membrane (17), the cultured cells (16), and the separated apical (15) and basolateral (18) compartments.

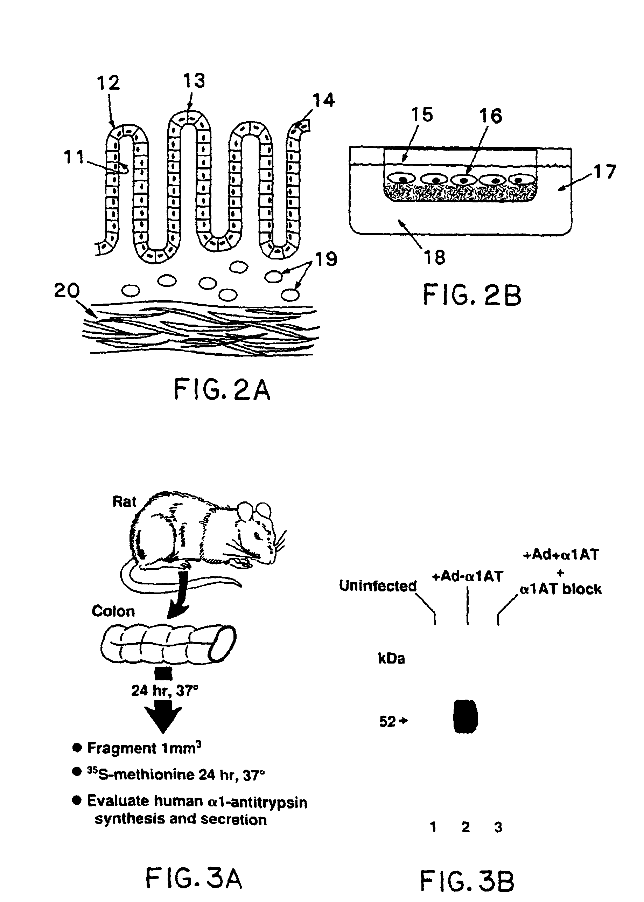

Figure 3. Demonstration of de novo synthesis and secretion of human α1-antitrypsin (α1AT) by rat colon exposed to the recombinant adenovirus Ad-α1AT ex vivo.

### DETAILED DESCRIPTION OF THE INVENTION

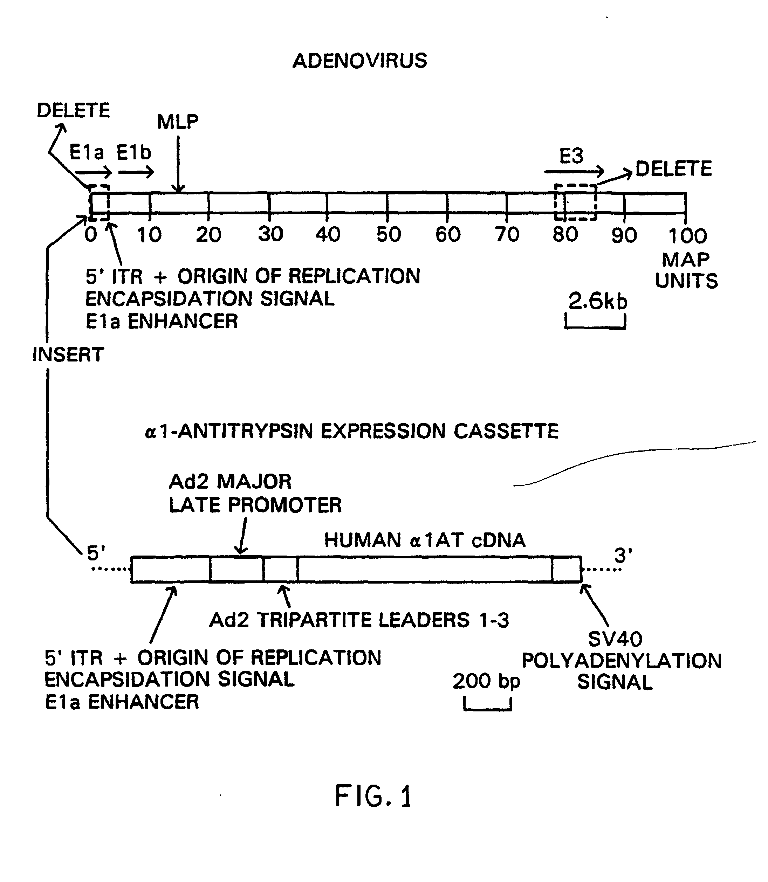

The present invention relates to the production of therapeutic proteins in the gastrointestinal tract. A replication deficient adenovirus (referred to below as the "modified adenovirus") is constructed with the coding sequences of the protein of therapeutic interest. As an example, the adenovirus Ad-α1AT containing the coding sequence of the human alAT gene is used (see Figure 1 and Rosenfeld M et al. (1991) Science 252:431-434). The modified adenovirus and no other virus, is placed into an enteric capsule (or alternatively, administered via tube past the stomach, orally or by tube into the stomach after the stomach lining fluid has been modified such that the virus will not be altered; or via the rectal route). Alternatively, a special coating may be applied to the adenovirus to prevent release and absorption of the modified adenovirus until the tablet reaches the basic (pH) environment of the duodenum, jejunum, ileum, or colon. For oral administration a capsule tablet or pill are convenient delivery vehicles; for rectal administration, a suppository may be preferred. Administration is then effected. The following scenario then occurs: (1) the cells (preferably, the epithelial cells) of the gastrointestinal tract are infected by the modified adenovirus; (2) the recombinant gene in the modified adenovirus sequence directs the synthesis of the recombinant protein which (depending upon how the sequences in the recombinant gene are engineered) can then be secreted into the circulation, into the lumen of the gastrointestinal tract or into both the circulation and the lumen of the gastrointestinal tract or within the epithelial cells of the gastrointestinal tract or in the local environs within the wall of the gastrointestinal tract; and (3) the therapeutic protein is then available to act systemically (when secreted into the circulation) or in the intestine (when secreted into the lumen), within the cells and/or the extracellular matrix of the wall of the gastrointestinal tract.

The present invention provides a practical, easy and safe way to administer recombinant proteins to humans. By using a replication deficient adenovirus, the process is safe because the virus cannot replicate in the target cells. By using a recombinant adenovirus, the target cells will produce the human therapeutic protein. By choosing the epithelium of the gastrointestinal tract for the target of infection by the replication deficient recombinant adenovirus, the invention permits ease of administration (preferably, by oral route via enteric coated capsule) via a route that can be used repetitively (for example, daily, or less frequently, depending on the chronicity of the recombinant adenovirus infection in the epithelial cells) and safely.

Because the epithelial cells of the gastrointestinal tract will secrete some of the product of the recombinant adenovirus through the basolateral surface of the infected epithelial cells, the invention is available for applications requiring systemic use. Because these epithelial cells also secrete some of the product through their apical surface, the invention is available for applications requiring luminal use (for example, intraluminal gastrointestinal disorders and gastrointestinal cancer). If the recombinant adenovirus is designed appropriately, the therapeutic protein will be available for therapeutic use within the epithelial cells of the gastrointestinal tract or in the local environs of the wall of the gastrointestinal tract (for example, for gastrointestinal tract cancer or gastrointestinal inflammatory disorders).

For disorders requiring systemic administration, this approach provides an easy and safe manner of administering recombinant protein to the circulation. Examples of such proteins include, but are not limited to:
- α1-antitrypsin - for α1-antitrypsin deficiency
- factor VIII - for hemophilia
- other coagulation factors - for bleeding disorders
- growth hormone - for growth disorders
- insulin - for diabetes
- other peptide hormones
- other pituitary hormones [adrenal cortical stimulating hormone (ACTH) and thyroid stimulating hormone (TSH) are just two examples]
- other lymphokines and cytokines for systemic therapy
- interferon γ - for granulomatous disease of childhood (and other diseases being investigated)
- interferon α - for leukemia and chronic active hepatitis
- erythropoietin - for chronic renal failure and other marrow suppressive disorders
- other hematologic growth factors - for marrow suppressive disorders
- administration e.g., tissue plasminogen activator for prevention of thrombosis in the pulmonary coronary arteries following reperfusion therapy, especially after balloon catheterization, or CD4 for human immunodeficiency virus (HIV) infection, and other recombinant proteins requiring systemic administration, whether short term or long term
- recombinant proteins for other hereditary disorders such as cerebrosidase deficiency and adenosine deaminase deficiency
- receptor agonists or antagonists - for example, for the control of systemic hypertension; interleukin-1 receptor antagonist for septic shock, rheumatoid arthritis and other disorders
- binding proteins for cytokines, lymphokines, and hormones - for example, tumor necrosis factor binding protein (a portion of the tumor necrosis factor receptor) for the treatment of shock and wasting disorders mediated by tumor necrosis factor

For hereditary and acquired disorders of the gastrointestinal tract, this approach provides a means of administering recombinant proteins to the surface or within the cells or extracellular matrix of the walls of the gastrointestinal tract. Examples of possible applications include:
- pancreatic enzymes for pancreatic deficiency disorders such as cystic fibrosis
- lactase for lactose intolerance and the appropriate enzymes for the small intestine disaccharidase deficiencies
- local therapy for gastrointestinal cancers with cytokines, tumor suppressor proteins (for example, p53 and retinoblastoma genes), and cytotoxic proteins
- prevention of cancer in individuals prone to gastrointestinal tract cancer (e.g., familial polyposis) with tumor suppressor proteins (for example, p53 and retinoblastoma genes).

For mammals and birds (more specifically, farm animals, for example - pigs, cattle, sheep, horses, dogs, cats, and chickens), this approach provides a means of administering recombinant proteins (for example, growth hormone) to these animals for the purposes of augmenting growth, generating characteristics for commercial purposes, and/or for general therapeutic purposes and for producing proteins from purified fractions given to humans, as well as antibodies for reagents reactive with human protein.

The use of proteins and polypeptides as therapeutic agents is greatly expanded according to the present invention by providing a means for delivering effective amounts of biologically active protein to a recipient individual. The preparations of this invention are suitably administered to animals, which include but are not limited to mammals (including humans), fish, and avians. The preparations are preferably administered to livestock (including cattle, horses, swine, sheep, goats, etc.), household pets (cats, dogs, canaries, parakeets, etc.) fish (especially in an aquarium or aquaculture environment, e.g., tropical fish, goldfish and other ornamental carp, catfish, trout, salmon, etc.) and avians, especially poultry such as chickens, ducks, geese, etc.

In one embodiment of the present invention, the replication-deficient adenovirus can be employed with animal feeds (or, with less dosage control, with animal drinking water) acting as a nontoxic, pharmaceutically acceptable carrier for administration to animals, e.g., livestock, household pets, fish, poultry, etc. In one aspect, this embodiment is useful for producing proteins for purification to human sera and to produce antibodies for reagents against human proteins in a different species, e.g., cattle, horse, sheep, goat, rabbit, swine, etc. In another aspect, this embodiment is useful in the treatment of disorders in which proteins or polypeptides are useful therapeutic agents, particularly when the gene coding the therapeutic protein is derived from the species being treated, or with sequences which are closely homologous to prevent immune reactions.

The replication-deficient adenovirus of the present invention can likewise be employed in admixture with conventional excipients, i.e., pharmaceutically acceptable organic or inorganic carrier substances suitable for enteral (e.g., oral) application that do not deleteriously react with the virus. Suitable pharmaceutically acceptable carriers are well known in the art. (Suitable vehicles include those that are acid resistant and base sensitive, that is, sufficiently so such that transport can be effected through the stomach without unacceptable degradation.) They include but are not limited to water, salt solutions, alcohols, gum arabic, vegetable oils, benzyl alcohols, polyethylene glycols, gelatine, carbohydrates such as lactose, anylose or starch, magnesium stearate, talc, silicic acid, viscous paraffin, perfume oil, fatty acid, monoglycerides and diglycerides, pentacrythritol fatty acid esters, hydroxy methyl cellulose, polyvinyl pyrrolidone, etc. Taking appropriate precautions not to kill the replication-deficient adenovirus, the preparations can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like that do not deleteriously react with the active virus. For example, agents can be used to increase the pH of the stomach, either directly (such as by buffers or bases) or indirectly (such as by drugs), to allow the virus to more readily pass through unharmed. They also can be combined where desired with other biologically active agents, e.g., antisense DNA or mRNA.

In another embodiment of the present invention, the replication-deficient adenovirus can be employed as a vaccine to develop immunity against infectious agents. The strategy is as follows. The gene coding for the protein against which immunity is to be developed is cloned into the replication deficient adenovirus construct; next, the replication deficient adenovirus containing the gene of interest is then administered to an animal (preferably, a human) as described herein. The gene sequences are designed such that the protein is secreted by the epithelial cells of the gastrointestinal tract into the systemic circulation such that immunity against the foreign proteins is developed.

Examples of this approach include the development of immunity against the hepatitis viruses, human immunodeficiency virus, and all other viruses that cause animal disease (particularly human disease). This strategy may also be used to develop immunity against bacteria, fungi, and other infectious agents.

A particularly interesting aspect of the present invention involves the use of replication-deficient adenovirus as a delivery system for chemotherapeutic agents, including antisense compounds, especially for use in cancer chemotherapy. Use with conventional chemotherapeutic agents is as discussed above. Briefly, use with antisense compounds for use against tumor cells in the bowel involves selecting mRNA as the primary drug target, with either another mRNA molecule or a synthetic oligo deoxynucleotide having the complementary base sequence to the mRNA forming a hybrid duplex by hydrogen-bonded base pairing. This hybridization can prevent expression of the target mRNA's protein product, a process called "translation arrest". Inhibition of mRNA is more efficient than inhibition of an enzyme active site because a single mRNA molecule gives rise to multiple protein copies. Thus, the selective inhibition of expression of a gene product required for cellular function yields the elusive but highly desired goal of chemotherapy: selective cell death. Such approaches are known in the literature, e.g., see J.S. Cohen, "Antisense Oligonucleotides as an Approach Toward Anti-Aids Therapy" at pages 195-224 in Design of Anti-Aids Drugs, E. deClerg (Ed), Elsevier Publishing Co. (1990); and S.L. Loke, et al. Current Topics in Microbiology and Immunology 141: 282-289 (1988).

For enternal applications, particularly suitable are tablets, dragees, liquids, drops, suppositories, or capsules, A syrup, elixir, or the like can be used wherein a sweetened vehicle is employed. Sustained or directed release compositions can be formulated, e.g., liposomes or those wherein the active virus is protected with differentially degradable coatings, e.g., by microencapsulation, multiple coatings, etc. It is also possible to freeze-dry the compositions and use the lyophilizates obtained.

Generally, the preparations of this invention are dispensed in unit dosage form comprising 10⁶ - 10¹⁴ pfu/ml of the replication-deficient adenovirus in a pharmaceutically acceptable carrier per unit dosage, preferably about 10¹⁰ - 10¹² pfu/ml. The dosages of the biologically active compounds administered according to this invention are generally known in the art but will frequently be reduced because of the improved delivery system provided by the present invention.

The actual preferred amounts of replication-deficient adenovirus administered in a specific case will vary according with the specific protein or polypeptide being utilized, the particular compositions formulated, the mode of application, and the particular situs and organism being treated.

The particular formulation employed will be selected according to conventional knowledge depending on the properties of the protein or polypeptide and the desired site of action to ensure bioavailability of the active ingredients, i.e., the extent to which the drug reaches its site of action or a biological fluid from which the drug has access to its site of action. Dosages for a given host can be determined using conventional considerations, e.g., by customary comparison of the differential activities of the subject preparations and a known appropriate, conventional pharmacological protocol.

The present invention is described in further detail in the following non-limiting examples.

### EXAMPLES

To demonstrate the feasibility of the invention, the replication deficient recombinant adenovirus Ad-α1AT (Figure 1) was used to transfer the sequences coding for human α1-antitrypsin to the epithelial cells of the colon. Three models were used: (1) T84 human colon carcinoma cells in vitro; (2) intact rat colon ex vivo; and (3) cotton rat colon in vivo.

The following protocols and experimental details are referenced in the Examples that follow:

The recombinant vector (Ad-α1AT) is constructed by deleting the majority of the E3 region and 2.6 mu from the left end of Ad5, and adding to the left end the α1-antitrypsin (α1AT) expression cassette from the plasmid pMLP-α1AT, containing regulatory sequences and a recombinant human alAT gene (Figure 1). Ad5 is commercially available from the American Type Culture Collection, Rockville, Maryland, USA. The methods for generating the α₁AT cDNA, the expression cassette, and the final vector adenovirus are prepared using the methods described by M. Rosenfeld, et al. in Science 252: 431-434 (1991).

The bottom of Figure 1 presents details of the α1AT expression cassette. ITR, inverted terminal repeat. To construct the recombinant viral vector Ad-α1AT, the expression cassette was ligated with ClaI precut Ad-d1327 DNA (to remove a portion of the Ela region from Ad-d1327). The recombinant adenovirus DNA was transfected into the 293 cell line where it was replicated, encapsulated into an infectious virus, and isolated by plaque purification. Individual plaques were amplified by propagation in 293 cells and viral DNA extracted. The intactness of the DNA of the recombinant virus was confirmed prior to use by restriction fragment analysis and Southern hybridization. Stocks of Ad-α1AT were propagated and titered in 293 cells. The virus was released from infected cells 36 hours post-infection by 5 cycles of freeze/thawing. The Ad-α1AT was further purified using CsCl gradients (for further details see Rosenfeld M et al. (1991) Science 252:431-434).

Figure 3A presents a demonstration of de novo synthesis and secretion of human α1-antitrypsin (α1AT) by rat colon exposed to the recombinant adenovirus Ad-α1AT. The colon was washed, the end tied off to make a "sausage" 2 - 5 cm. in length, and 50 - 100 microliters of 10¹⁰ - 10¹² pfu/ml Ad-alphalAT in LHC-8 medium injected into the lumen. The "sausage" was then incubated for 24 hr. 37°, washed, cut into 1mm² fragments, and ³²S-methionine (500 mCi/ml) added in methionine-minus LHC-8 medium. After incubation for 24 hr, 37°, the fluid bathing the fragments were evaluated for the presence of human α1AT by immunoprecipitation, sodium dodecyl sulfate acrylamide gels and autoradiography. The results are set forth in Figure 3B, wherein: Lane 1 - uninfected colon; lane 2 - colon infected with Ad-α1AT; and lane 3 - same as lane 2, but with the antibody exposed to unlabeled human α1AT (to demonstrate the specificity of the antibody). The 52 kDa human alAT is indicated by the arrow.

### EXAMPLE 1

### T84 Human Colon Carcinoma Cells In Vitro

This model was used to demonstrate that human colon epithelial cells can be infected by Ad-α1AT, and that the infection resulted in the secretion of human α1AT to the apical surface (i.e., to the lumen side of the epithelium) and to the basolateral surface (i.e., the circulation side of the epithelium). To accomplish this, the T84 cell line was grown on microporous membranes until they became confluenent and formed tight junctions (electrical resistance >150 ohm-cm² across the epithelium). The microporous polycarbonate membrane (4.7 cm², pore size 3.0 um, Transwell Col., Coster, Cambridge, MA) with the epithelial cells separate two chambers that contain culture fluid, i.e., an in vitro system that mimics the epithelium in vivo. The upper chamber faces the apical surface and the lower chamber faces the basolateral surface. The combination of the cells and the tight junctions between the cells physically separate the fluids and the upper and lower chambers (equivalent to the in vivo situation where the apical surface abuts the inside lumen of the colon and the basolateral surface abuts the tissue side (and thus the circulation; see Figure 2). The cells are cultured in DMEM, 2% fetal calf serum for 1.5 hr, 37° and then in DMEM, 10% fetal calt serum for 24 hr, 37° with no adenovirus or with Ad-α1AT (from the apical side as would occur in vivo). Three different intensities of infection were used [measured in plaque-forming units (pfu), the number of infectious viral particles per ml of fluid; 5x10⁹, 10¹⁰, and 2.5x10¹⁰ pfu/culture]. The media was then collected and evaluated for the presence of human α1-antitrypsin using an enzyme-linked immunoassay (Wewers MD et al. (1987) N Engl J Med 316: 1055-1062). The data demonstrates that Ad-α1AT infection causes the human colon epithelial cells to secrete α1AT, and to do so in both directions, i.e., to the apical and basolateral surfaces. The amount secreted to the apical surface compared to the basolateral surface ranged from 3.98 to 4.69 (average 4.34) i.e., for every 4.34 molecules secreted into the lumen (where it would eventually be excreted in vivo), 1 molecule would be secreted into the tissue (where it would be available to the circulation).

The results of Example 1 are shown in Table I.

**Table I**

| Polarity of Secretion of Human α1-Antitrypsin by the Human T84 Colon Carcinoma Cell Line Following Infection by the Recombinant Adenovirus Ad-α1AT | | | |
|---|---|---|---|
| Infection¹ | Amount α1AT Secreted in 24 hr (µg)² | | Ratio of α1AT in Apical and Basolateral Compartments |
| | Apical | Basolateral | |
| None | 0 | 0 | --- |
| 5x10⁹ | 3.54 | 0.89 | 3.98 |
| 10¹⁰ | 7.42 | 1.58 | 4.69 |
| 2.5x10¹⁰ | 8.89 | 2.05 | 4.34 |

| | | | |
|---|---|---|---|
| ¹ number of pfu Ad-α1AT added to the culture; all cells were grown on 4.7 cm² microporous membranes until tight junctions were formed (electrical resistance > 150 ohm-cm²) | | | |
| ² Measured by enzyme linked immunoassay (Wewers MD et al. (1987) N Engl J Med 316: 1055-1062) | | | |

### EXAMPLE 2

### Intact Rat and Cotton Rat Colon In Vitro

This model was used to determine if the recombinant adenovirus can infect colon epithelial cells in circumstance where the cells were normal (i.e., not derived from a neoplasm as in the T84 model) and were in their normal architectural configuration. To do this, rat colon was removed, washed and a 2-3 cm section made into a closed "sausage" by tying off both ends (Figure 3). Ad-α1AT was injected into the lumen (e.g., equivalent to live recombinant adenovirus being released from enteric coated capsules). The "sausage" was placed in culture media for 24 hr, 37° and then evaluated in two ways.

First, the colon was fragmented into 1 mm³ pieces, ³⁵S-methionine was added, the culture continued for 24 hr, 37°, and the ability of the colon to de novo synthesize and secrete human α1AT evaluated using immunoprecipitation, sodium dodecyl sulfate acrylamide gels and autoradiography (see Rosenfeld M et al. (1991) Science 252:431-434 for details of the methods). The results demonstrate that uninfected rat colon does not synthesize and secrete human α1AT in vitro, but that Ad-α1AT infected rat colon does (Figure 3).

A similar technique was used to evaluate cotton rat colon, but using enzyme-linked immunoassay (ELISA) to quantify the amount of human α1AT secreted into the lumen (i.e., apical secretion; it is not possible to evaluate basolateral secretion in this model). Following 48 hr infection with approximately 10¹¹ pfu AdalAT injected into the lumen of cotton rat colon "sausage" in vitro, evaluation of the luminal fluid demonstrated 3.3 ± 0.6 µg/ml human α1AT.

### EXAMPLE 3

### Cotton Rat Colon In Vivo

This model was used to demonstrate that the concept will work in vivo in living animals. Two strategies were used, both in cotton rats. First, following general anesthesia and laparotomy, a section of colon was ligated in two places to form an in vivo "sausage" in a fashion that permitted normal blood flow to that segment. Ad-α1AT was injected into the lumen and the laparotomy closed. The animals were maintained without oral intake. After 48 hr, a serum sample was taken and evaluated for the presence of human α1AT by ELISA. Second, following general anesthesia and laparotomy 10¹⁰ - 10¹² pfu of, AdalAT was injected into the lumen of the colon without ligation. After 48 hr, a serum sample was taken and evaluated for human α1AT by ELISA. In both cases human α1AT was clearly evident.

The results are shown in Table II.

**Table II**

| Serum Levels of Human α1-antitrypsin in Cotton Rats 48 hr Following In Vivo Administration of Ad-α1AT in the Lumen of the colon | |
|---|---|
| Condition | Serum α1AT Level (ng/ml)³ |
| Mock infection | 0 |
| "Sausage" infection¹ | 145 ± 29 |
| Direct infection² | 74 ± 9 |

| | |
|---|---|
| ¹ Ad-α1AT (50-100 µl, approximately 10¹¹ pfu) injected into the lumen of a segment of bowel isolated by ligation at both ends. | |
| ² Similar to the "sausage" infection, but without isolating a segment of bowel by ligation. | |
| ³ Measured by enzyme linked immunoassay (Wewers MD et al. (1987) N Engl J Med 316: 1055-1062). | |

## Claims (Claims for the following Contracting State(s): AT, BE, CH, LI, DE, DK, FR, GB, IT, LU, NL, SE)

1. Use of a replication deficient adenovirus comprising a DNA segment encoding a biologically active therapeutic protein in the manufacture of a medicament for treatment, by administration to the gastrointestinal tract of an animal for production of the protein in the gastrointestinal tract, of a disorder wherein the protein has a therapeutic effect, which medicament contains no virus other than said replication deficient adenovirus.

2. Use of a composition comprising a replication deficient adenovirus comprising a DNA segment encoding a biologically active protein and no virus other than said replication deficient adenovirus, for production of the protein in the gastrointestinal tract of an animal for a non-therapeutic and non-diagnostic purpose.

3. The use according to Claim 1 or Claim 2 wherein the adenovirus is encapsidated in an enteric capsule.

4. The use according to any of Claims 1-3 wherein the protein is a coagulation factor, a pituitary hormone, a peptide hormone, a cytokine, a tumor suppressor protein, a hematologic growth factor, a receptor agonist, or a receptor antagonist.

5. The use according to any of Claims 1-3 wherein the protein is α1-antitrypsin, erythropoietin, Factor VIII, growth hormone, tumor necrosis binding protein, interleukin-1 receptor antagonist, interferon γ, interferon α, or insulin.

6. The use according to any of Claims 1-3 wherein the adenovirus is Ad-α1AT.

7. The use according to any one of the preceding claims wherein the animal is a mammal, avian, or fish.

8. The use according to Claim 7 wherein the animal is a mammal.

9. The use according to claim 7 wherein the animal is a human.

10. The use according to Claim 7 wherein the animal is a pig, sheep, cattle, horse, cat, or dog.

11. The use according to Claim 7 wherein the animal is a chicken.

12. The use according to any one of the preceding claims wherein the animal develops immunity against the protein following production of the protein.

13. An enteric capsule comprising a replication-deficient adenovirus containing a DNA segment encoding a therapeutic protein and no other virus that can foster replication of said adenovirus.

14. An enteric capsule according to Claim 13 wherein the protein is a therapeutic protein.

15. An enteric capsule according to Claim 13 wherein the protein is a coagulation factor, a pituitary hormone, a peptide hormone, a cytokine, a tumor suppressor protein, a hematologic growth factor, a receptor agonist, or a receptor antagonist.

16. An enteric capsule according to Claim 13 wherein the protein is α1-antitrypsin, erythropoietin, Factor VIII, growth hormone, tumor necrosis binding protein, interleukin-1 receptor antagonist, interferon y, interferon α, or insulin.

17. An enteric capsule according to Claim 13 wherein the adenovirus is Ad-α1AT.

18. A pharmaceutical composition comprising:
a replication deficient adenovirus containing at least one DNA segment encoding a therapeutic protein, said adenovirus contained in a vehicle for delivery to the gastro-intestinal tract that is acid-resistant and base-sensitive, and no other virus that can foster replication of said adenovirus, and
a pharmaceutically acceptable diluent, carrier, or excipient.

19. A pharmaceutical composition according to Claim 18 wherein the adenovirus is contained in an enteric capsule.

20. A pharmaceutical composition according to Claim 18 or Claim 20 wherein the protein is a therapeutic protein.

21. A pharmaceutical composition according to Claim 18 or Claim 19 wherein the protein is a coagulation factor, a pituitary hormone, a peptide hormone, a cytokine, a tumor suppressor protein, a hematologic growth factor, a receptor agonist, or a receptor antagonist.

22. A pharmaceutical composition according to Claim 18 or Claim 19 wherein the protein is α1-antitrypsin, erythropoietin, Factor VIII, growth hormone, tumor necrosis binding protein, interleukin-1 receptor antagonist, interferon γ, interferon α, or insulin.

23. A pharmaceutical composition according to Claim 18 or Claim 19 wherein the adenovirus is Ad-α1AT.

## Claims (Claims for the following Contracting State(s): ES, GR)

1. Use of a replication deficient adenovirus comprising a DNA segment encoding a biologically active therapeutic protein in the manufacture of a medicament for treatment, by administration to the gastrointestinal tract of an animal for production of the protein in the gastrointestinal tract, of a disorder wherein the protein has a therapeutic effect, which medicament contains no virus other than said replication deficient adenovirus.

2. Use of a composition comprising a replication deficient adenovirus comprising a DNA segment encoding a biologically active protein and no virus other than said replication deficient adenovirus, for production of the protein in the gastrointestinal tract of an animal for a non-therapeutic and non-diagnostic purpose.

3. The use according to Claim 1 or Claim 2 wherein the adenovirus is encapsidated in an enteric capsule.

4. The use according to any of Claims 1-3 wherein the protein is a coagulation factor, a pituitary hormone, a peptide hormone, a cytokine, a tumor suppressor protein, a hematologic growth factor, a receptor agonist, or a receptor antagonist.

5. The use according to any of Claims 1-3 wherein the protein is α1-antitrypsin, erythropoietin, Factor VIII, growth hormone, tumor necrosis binding protein, interleukin-1 receptor antagonist, interferon γ, interferon α, or insulin.

6. The use according to any of Claims 1-3 wherein the adenovirus is Ad-α1AT.

7. The use according to any one of the preceding claims wherein the animal is a mammal, avian, or fish.

8. The use according to Claim 7 wherein the animal is a mammal.

9. The use according to claim 7 wherein the animal is a human.

10. The use according to Claim 7 wherein the animal is a pig, sheep, cattle, horse, cat, or dog.

11. The use according to Claim 7 wherein the animal is a chicken.

12. The use according to any one of the preceding claims wherein the animal develops immunity against the protein following production of the protein.

13. A process for the manufacture of an enteric capsule comprising a replication-deficient adenovirus containing a DNA segment encoding a therapeutic protein and no other virus that can foster replication of said adenovirus.

14. A process for the manufacture of an enteric capsule according to Claim 13 wherein the protein is a therapeutic protein.

15. A process for the manufacture of an enteric capsule according to Claim 13 wherein the protein is a coagulation factor, a pituitary hormone, a peptide hormone, a cytokine, a tumor suppressor protein, a hematologic growth factor, a receptor agonist, or a receptor antagonist.

16. A process for the manufacture of an enteric capsule according to Claim 13 wherein the protein is α1-antitrypsin, erythropoietin, Factor VIII, growth hormone, tumor necrosis binding protein, interleukin-1 receptor antagonist, interferon γ, interferon α, or insulin.

17. A process for the manufacture of an enteric capsule according to Claim 13 wherein the adenovirus is Ad-α1AT.

18. A process for the manufacture of a pharmaceutical composition comprising:
a replication deficient adenovirus containing at least one DNA segment encoding a therapeutic protein, said adenovirus contained in a vehicle for delivery to the group-intestinal tract that is acid-resistant and base-sensitive, and no other virus that can foster replication of said adenovirus, and
a pharmaceutically acceptable diluent, carrier, or excipient.

19. A process for the manufacture of a pharmaceutical composition according to Claim 18 wherein the adenovirus is contained in an enteric capsule.

20. A process for the manufacture of a pharmaceutical composition according to Claim 18 or Claim 20 wherein the protein is a therapeutic protein.

21. A process for the manufacture of a pharmaceutical composition according to Claim 18 or Claim 19 wherein the protein is a coagulation factor, a pituitary hormone, a peptide hormone, a cytokine, a tumor suppressor protein, a hematologic growth factor, a receptor, agonist, or a receptor antagonist.

22. A process for the manufacture of a pharmaceutical composition according to Claim 18 or Claim 19 wherein the protein is α1-antitrypsin, erythropoietin, Factor VIII, growth hormone, tumor necrosis binding protein, interleukin-1 receptor antagonist, interferon γ, interferon α, or insulin.

23. A process for the manufacture of a pharmaceutical composition according to Claim 18 or Claim 19 wherein the adenovirus is Ad-α1AT.

## Patentansprüche (Patentansprüche für folgende(n) Vertragsstaat(en): AT, BE, CH, LI, DE, DK, FR, GB, IT, LU, NL, SE)

1. Verwendung eines replikationsdefizienten Adenovirus, das ein DNA-Segment umfasst, das für ein biologisch aktives therapeutisches Protein kodiert, bei der Herstellung eines Medikaments zur Behandlung einer Störung, worin das Protein therapeutische Wirkung aufweist, durch Verabreichung an den Magen-Darm-Trakt eines Tieres zur Produktion des Proteins im Magen-Darm-Trakt, wobei das Medikament kein anderes Virus als das replikationsdefiziente Adenovirus enthält.

2. Verwendung einer Zusammensetzung, die ein replikationsdefizientes Adenovirus, das ein für ein biologisch aktives Protein kodierendes DNA-Segment kodiert, und kein anderes Virus als das replikationsdefiziente Adenovirus umfasst, zur Produktion des Proteins im Magen-Darm-Trakt eines Tieres für nichttherapeutische und nichtdiagnostische Zwecke.

3. Verwendung nach Anspruch 1 oder 2, worin das Adenovirus in einer enterischen Kapsel eingekapselt ist.

4. Verwendung nach einem der Ansprüche 1 bis 3, worin das Protein ein Gerinnungsfaktor, ein Hypophysenhormon, ein Peptidhormon, ein Zytokin, ein Tumorsuppressorprotein, ein hämatologischer Wachstumsfaktor, ein Rezeptoragonist oder ein Rezeptorantagonist ist.

5. Verwendung nach einem der Ansprüche 1 bis 3, worin das Protein α1-Antitrypsin, Erythropoietin, Faktor VIII, Wachstumshormon, Tumornekrosefaktor-Bindungsprotein, Interleukin-1-Rezeptorantagonist, Interferon-γ, Interferon-α oder Insulin ist.

6. Verwendung nach einem der Ansprüche 1 bis 3, worin das Adenovirus Ad-α1AT ist.

7. Verwendung nach einem der vorangegangenen Ansprüche, worin das Tier ein Säugetier, Vogel oder Fisch ist.

8. Verwendung nach Anspruch 7, worin das Tier ein Säugetier ist.

9. Verwendung nach Anspruch 7, worin das Tier ein Mensch ist.

10. Verwendung nach Anspruch 7, worin das Tier ein Schwein, Schaf, Rind, Pferd, eine Katze oder ein Hund ist.

11. Verwendung nach Anspruch 7, worin das Tier ein Huhn ist.

12. Verwendung nach einem der vorangegangenen Ansprüche, worin das Tier nach der Produktion des Proteins Immunität gegen das Protein entwickelt.

13. Enterische Kapsel, die ein replikationsdefizientes Adenovirus, das ein für ein therapeutisches Protein kodierendes DNA-Segment enthält, und kein anderes Virus umfasst, das die Replikation des Adenovirus fördern kann.

14. Enterische Kapsel nach Anspruch 13, worin das Protein ein therapeutisches Protein ist.

15. Enterische Kapsel nach Anspruch 13, worin das Protein ein Gerinnungsfaktor, ein Hypophysenhormon, ein Peptidhormon, ein Zytokin, ein Tumorsuppressorprotein, ein hämatologischer Wachstumsfaktor, ein Rezeptoragonist oder ein Rezeptorantagonist ist.

16. Enterische Kapsel nach Anspruch 13, worin das Protein α1-Antitrypsin, Erythropoietin, Faktor VIII, Wachstumshormon, Tumornekrosefaktor-Bindungsprotein, Interleukin-1-Rezeptorantagonist, Interferon-γ, Interferon-α oder Insulin ist.

17. Enterische Kapsel nach Anspruch 13, worin das Adenovirus Ad-α1AT ist.

18. Pharmazeutische Zusammensetzung, Folgendes umfassend:
ein replikationsdefizientes Adenovirus, das zumindest ein für ein therapeutisches Protein kodierendes DNA-Segment enthält, wobei das Adenovirus in einem säurebeständigen und basenempfindlichen Vehikel zur Abgabe an den Magen-Darm-Trakt enthalten ist, und kein anderes Virus, das die Replikation des Adenovirus fördern kann, sowie
einen pharmazeutisch annehmbaren Verdünner, Träger oder Exzipienten.

19. Pharmazeutische Zusammensetzung nach Anspruch 18, worin das Adenovirus in einer enterischen Kapsel enthalten ist.

20. Pharmazeutische Zusammensetzung nach Anspruch 18 oder 19, worin das Protein ein therapeutisches Protein ist.

21. Pharmazeutische Zusammensetzung nach Anspruch 18 oder 19, worin das Protein ein Gerinnungsfaktor, ein Hypophysenhormon, ein Peptidhormon, ein Zytokin, ein Tumorsuppressorprotein, ein hämatologischer Wachstumsfaktor, ein Rezeptoragonist oder ein Rezeptorantagonist ist.

22. Pharmazeutische Zusammensetzung nach Anspruch 18 oder 19, worin das Protein α1-Antitrypsin, Erythropoietin, Faktor VIII, Wachstumshormon, Tumornekrosefaktor-Bindungsprotein, Interleukin-1-Rezeptorantagonist, Interferon-γ, Interferon-α oder Insulin ist.

23. Pharmazeutische Zusammensetzung nach Anspruch 18 oder 19, worin das Adenovirus Ad-α1AT ist.

## Patentansprüche (Patentansprüche für folgende(n) Vertragsstaat(en): ES, GR)

1. Verwendung eines replikatiorisdefizienten Adenovirus, das ein DNA-Segment umfasst, das für ein biologisch aktives therapeutisches Protein kodiert, bei der Herstellung eines Medikaments zur Behandlung einer Störung, worin das Protein therapeutische Wirkung aufweist, durch Verabreichung an den Magen-Darm-Trakt eines Tieres zur Produktion des Proteins im Magen-Darm-Trakt, wobei das Medikament kein anderes Virus als das replikationsdefiziente Adenovirus enthält.

2. Verwendung einer Zusammensetzung, die ein replikationsdefizientes Adenovirus, das ein für ein biologisch aktives Protein kodierendes DNA-Segment kodiert, und kein anderes Virus als das replikationsdefiziente Adenovirus umfasst, zur Produktion des Proteins im Magen-Darm-Trakt eines Tieres für nichttherapeutische und nichtdiagnostische Zwecke.

3. Verwendung nach Anspruch 1 oder 2, worin das Adenovirus in einer enterischen Kapsel eingekapselt ist.

4. Verwendung nach einem der Ansprüche 1 bis 3, worin das Protein ein Gerinnungsfaktor, ein Hypophysenhormon, ein Peptidhormon, ein Zytokin, ein Tumorsuppressorprotein, ein hämatologischer Wachstumsfaktor, ein Rezeptoragonist oder ein Rezeptorantagonist ist.

5. Verwendung nach einem der Ansprüche 1 bis 3, worin das Protein α1-Antitrypsin, Erythropoietin, Faktor VIII, Wachstumshormon, Tumornekrosefaktor-Bindungsprotein, Interteukin-1-Rezeptorantagonist, Interferon-γ, Interferon-α oder Insulin ist.

6. Verwendung nach einem der Ansprüche 1 bis 3, worin das Adenovirus Ad-α1AT ist.

7. Verwendung nach einem der vorangegangenen Ansprüche, worin das Tier ein Säugetier, Vogel oder Fisch ist.

8. Verwendung nach Anspruch 7, worin das Tier ein Säugetier ist.

9. Verwendung nach Anspruch 7, worin das Tier ein Mensch ist.

10. Verwendung nach Anspruch 7, worin das Tier ein Schwein, Schaf, Rind, Pferd, eine Katze oder ein Hund ist.

11. Verwendung nach Anspruch 7, worin das Tier ein Huhn ist.

12. Verwendung nach einem der vorangegangenen Ansprüche, worin das Tier nach der Produktion des Proteins Immunität gegen das Protein entwickelt.

13. Verfahren zur Herstellung einer enterischen Kapsel, die ein replikationsdefizientes Adenovirus, das ein für ein therapeutisches Protein kodierendes DNA-Segment enthält, und kein anderes Virus umfasst, das die Replikation des Adenovirus fördern kann.

14. Verfahren zur Herstellung einer enterischen Kapsel nach Anspruch 13, worin das Protein ein therapeutisches Protein ist.

15. Verfahren zur Herstellung einer enterischen Kapsel nach Anspruch 13, worin das Protein ein Gerinnungsfaktor, ein Hypophysenhormon, ein Peptidhormon, ein Zytokin, ein Tumorsuppressorprotein, ein hämatologischer Wachstumsfaktor, ein Rezeptoragonist oder ein Rezeptorantagonist ist.

16. Verfahren zur Herstellung einer enterischen Kapsel nach Anspruch 13, worin das Protein α1-Antitrypsin, Erythropoietin, Faktor VIII, Wachstumshormon, Tumornekrosefaktor-Bindungsprotein, Interleukin-1-Rezeptorantagonist, Interferon-γ, Interferon-α oder Insulin ist.

17. Verfahren zur Herstellung einer enterischen Kapsel nach Anspruch 13, worin das Adenovirus Ad-α1AT ist.

18. Verfahren zur Herstellung einer pharmazeutischen Zusammensetzung, Folgendes umfassend:
ein replikationsdefizientes Adenovirus, das zumindest ein für ein therapeutisches Protein kodierendes DNA-Segment enthält, wobei das Adenovirus in einem säurebeständigen und basenempfindlichen Vehikel zur Abgabe an den Magen-Darm-Trakt enthalten ist, und kein anderes Virus, das die Replikation des Adenovirus fördern kann, sowie
einen pharmazeutisch annehmbaren Verdünner, Träger oder Exzipienten.

19. Verfahren zur Herstellung einer pharmazeutischen Zusammensetzung nach Anspruch 18, worin das Adenovirus in einer enterischen Kapsel enthalten ist.

20. Verfahren zur Herstellung einer pharmazeutischen Zusammensetzung nach Anspruch 18 oder 19, worin das Protein ein therapeutisches Protein ist.

21. Verfahren zur Herstellung einer pharmazeutischen Zusammensetzung nach Anspruch 18 oder 19, worin das Protein ein Gerinnungsfaktor, ein Hypophysenhormon, ein Peptidhormon, ein Zytokin, ein Tumorsuppressorprotein, ein hämatologischer Wachstumsfaktor, ein Rezeptoragonist oder ein Rezeptorantagonist ist.

22. Verfahren zur Herstellung einer pharmazeutischen Zusammensetzung nach Anspruch 18 oder 19, worin das Protein α1-Antitrypsin, Erythropoietin, Faktor VIII, Wachstumshormon, Tumornekrosefaktor-Bindungsprotein, Interleukin-1-Rezeptorantagonist, Interferon-γ, Interferon-α oder Insulin ist.

23. Verfahren zur Herstellung einer pharmazeutischen Zusammensetzung nach Anspruch 18 oder 19, worin das Adenovirus Ad-α1AT ist.

## Revendications (Revendications pour l'(les) Etat(s) contractant(s) suivant(s): AT, BE, CH, LI, DE, DK, FR, GB, IT, LU, NL, SE)

1. Utilisation d'un adénovirus déficient en réplication comprenant un segment d'ADN codant pour une protéine thérapeutique biologiquement active dans la fabrication d'un médicament pour le traitement, par administration à la voie gastro-intestinale d'un animal pour la production de la protéine dans la voie gastro-intestinale, d'un trouble où la protéine a un effet thérapeutique, lequel médicament ne contient pas de virus autre que ledit adénovirus déficient en réplication.

2. Utilisation d'une composition comprenant un adénovirus déficient en réplication comprenant un segment d'ADN codant pour une protéine biologiquement active et pas de virus autre que ledit adénovirus déficient en réplication, pour la production de la protéine dans la voie gastro-intestinale d'un animal dans un but non thérapeutique et non diagnostique.

3. Utilisation selon la revendication 1 ou la revendication 2, où l'adénovirus est encapsidé dans une capsule entérique.

4. Utilisation selon l'une quelconque des revendications 1-3, où la protéine est un facteur de coagulation, une hormone pituitaire, une hormone peptidique, une cytokine, une protéine suppresseur de tumeur, un facteur de croissance hématologique, un agoniste de récepteur ou un antagoniste de récepteur.

5. Utilisation selon l'une quelconque des revendications 1-3, où la protéine est l'α1-antitrypsine, l'érythropoïétine, le Facteur VIII, l'hormone de croissance, la protéine de liaison de la nécrose de la tumeur, l'antagoniste du récepteur d'interleukine 1, l'interféron γ, l'interféron α ou l'insuline.

6. Utilisation selon l'une quelconque des revendications 1-3, où l'adénovirus est Ad-α1AT.

7. Utilisation selon l'une quelconque des revendications précédentes, où l'animal est un mammifère, un avien ou un poisson.

8. Utilisation selon la revendication 7, où l'animal est un mammifère.

9. Utilisation selon la revendication 7, où l'animal est un humain.

10. Utilisation selon la revendication 7, où l'animal est un cochon, un mouton, un bovin, un cheval, un chat ou un chien.

11. Utilisation selon la revendication 7, où l'animal est un poussin.

12. Utilisation selon l'une quelconque des revendications précédentes, où l'animal développe une immunité contre la protéine à la suite de la production de la protéine.

13. Capsule entérique comprenant un adénovirus déficient en réplication contenant un segment d'ADN codant pour une protéine thérapeutique et pas d'autre virus qui peut favoriser la réplication dudit adénovirus.

14. Capsule entérique selon la revendication 13, où la protéine est une protéine thérapeutique.

15. Capsule entérique selon la revendication 13, où la protéine est un facteur de coagulation, une hormone pituitaire, une hormone peptidique, une cytokine, une protéine suppresseur de tumeur, un facteur de croissance hématologique, un agoniste de récepteur ou un antagoniste de récepteur.

16. Capsule entérique selon la revendication 13, où la protéine est l'α1-antitrypsine, l'érythropoïétine, le Facteur VIII, l'hormone de croissance, la protéine de liaison de la nécrose de tumeur, l'antagoniste du récepteur d'interleukine 1, l'interféron γ, l'interféron α ou l'insuline.

17. Capsule entérique selon la revendication 13, où l'adénovirus est Ad-α1AT.

18. Composition pharmaceutique comprenant :
un adénovirus déficient en réplication contenant au moins un segment d'ADN codant pour une protéine thérapeutique, ledit adénovirus contenu dans un véhicule pour délivrance à la voie gastro-intestinale qui est résistant aux acides et sensible aux bases et pas d'autre virus qui peut favoriser la réplication dudit adénovirus, et
un diluant, support ou excipient pharmaceutiquement acceptable.

19. Composition pharmaceutique selon la revendication 18, où l'adénovirus est contenu dans une capsule entérique.

20. Composition pharmaceutique selon la revendication 18 ou la revendication 20, où la protéine est une protéine thérapeutique.

21. Composition pharmaceutique selon la revendication 18 ou la revendication 19, où la protéine est un facteur de coagulation, une hormone pituitaire, une hormone peptidique, une cytokine, une protéine suppresseur de tumeur, un facteur de croissance hématologique, un agoniste de récepteur ou un antagoniste de récepteur.

22. Composition pharmaceutique selon la revendication 18 ou la revendication 19, où la protéine est l'α1-antitrypsine, l'érythropoïétine, le Facteur VIII, l'hormone de croissance, la protéine de liaison de la nécrose de la tumeur, l'antagoniste du récepteur d'interleukine-1, l'interféron γ, l'interféron α ou l'insuline.

23. Composition pharmaceutique selon la revendication 18 ou la revendication 19, où l'adénovirus est Ad-α1AT.

## Revendications (Revendications pour l'(les) Etat(s) contractant(s) suivant(s): ES, GR)

1. Utilisation d'un adénovirus déficient en réplication comprenant un segment d'ADN codant pour une protéine thérapeutique biologiquement active dans la fabrication d'un médicament pour le traitement, par administration à la voie gastro-intestinale d'un animal pour la production de la protéine dans la voie gastro-intestinale, d'un trouble où la protéine a un effet thérapeutique, lequel médicament ne contient pas de virus autre que ledit adénovirus déficient en réplication.

2. Utilisation d'une composition comprenant un adénovirus déficient en réplication comprenant un segment d'ADN codant pour une protéine biologiquement active et pas de virus autre que ledit adénovirus déficient en réplication, pour la production de la protéine dans la voie gastro-intestinale d'un animal dans un but non thérapeutique et non diagnostique.

3. Utilisation selon la revendication 1 ou la revendication 2, où l'adénovirus est encapsidé dans une capsule entérique.

4. Utilisation selon l'une quelconque des revendications 1-3, où la protéine est un facteur de coagulation, une hormone pituitaire, une hormone peptidique, une cytokine, une protéine suppresseur de tumeur, un facteur de croissance hématologique, un agoniste de récepteur ou un antagoniste de récepteur.

5. Utilisation selon l'une quelconque des revendications 1-3, où la protéine est l'α1-antitrypsine, l'érythropoïétine, le Facteur VIII, l'hormone de croissance, la protéine de liaison de la nécrose de la tumeur, l'antagoniste du récepteur d'interleukine 1, l'interféron γ, l'interféron α ou l'insuline.

6. Utilisation selon l'une quelconque des revendications 1-3, où l'adénovirus est Ad-α1AT.

7. Utilisation selon l'une quelconque des revendications précédentes, où l'animal est un mammifère, un avien ou un poisson.

8. Utilisation selon la revendication 7, où l'animal est un mammifère.

9. Utilisation selon la revendication 7, où l'animal est un humain.

10. Utilisation selon la revendication 7, où l'animal est un cochon, un mouton, un bovin, un cheval, un chat ou un chien.

11. Utilisation selon la revendication 7, où l'animal est un poussin.

12. Utilisation selon l'une quelconque des revendications précédentes, où l'animal développe une immunité contre la protéine à la suite de la production de la protéine.

13. Capsule entérique comprenant un adénovirus déficient en réplication contenant un segment d'ADN codant pour une protéine thérapeutique et pas d'autre virus qui peut favoriser la réplication dudit adénovirus.

14. Procédé pour la fabrication d'une capsule entérique selon la revendication 13, où la protéine est une protéine thérapeutique.

15. Procédé pour la fabrication d'une capsule entérique selon la revendication 13, où la protéine est un facteur de coagulation, une hormone pituitaire, une hormone peptidique, une cytokine, une protéine suppresseur de tumeur, un facteur de croissance hématologique, un agoniste de récepteur ou un antagoniste de récepteur.

16. Procédé pour la fabrication d'une capsule entérique selon la revendication 13, où la protéine est l'α1-antitrypsine, l'érythropoïétine, le Facteur VIII, l'hormone de croissance, la protéine de liaison de la nécrose de tumeur, l'antagoniste du récepteur d'interleukine-1, l'interféron γ, l'interféron α ou l'insuline.

17. Procédé pour la fabrication d'une capsule entérique selon la revendication 13, où l'adénovirus est Ad-α1AT.

18. Procédé pour la fabrication d'une composition pharmaceutique comprenant :
un adénovirus déficient en réplication contenant au moins un segment d'ADN codant pour une protéine thérapeutique, ledit adénovirus contenu dans un véhicule pour délivrance à la voie gastro-intestinale qui est résistant aux acides et sensible aux bases et pas d'autre virus qui peut favoriser la réplication dudit adénovirus, et
un diluant, support ou excipient pharmaceutiquement acceptable.

19. Procédé pour la fabrication d'une composition pharmaceutique selon la revendication 18, où l'adénovirus est contenu dans une capsule entérique.

20. Procédé pour la fabrication d'une composition pharmaceutique selon la revendication 18 ou la revendication 20, où la protéine est une protéine thérapeutique.

21. Procédé pour la fabrication d'une composition pharmaceutique selon la revendication 18 ou la revendication 19, où la protéine est un facteur de coagulation, une hormone pituitaire, une hormone peptidique, une cytokine, une protéine suppresseur de tumeur, un facteur de croissance hématologique, un agoniste de récepteur ou un antagoniste de récepteur.

22. Procédé pour la fabrication d'une composition pharmaceutique selon la revendication 18 ou la revendication 19, où la protéine est l'α1-antitrypsine, l'érythropoïétine, le Facteur VIII, l'hormone de croissance, la protéine de liaison de la nécrose de la tumeur, l'antagoniste du récepteur d'interleukine 1, l'interféron γ, l'interféron α ou l'insuline.

23. Procédé pour la fabrication d'une composition pharmaceutique selon la revendication 18 ou la revendication 19, où l'adénovirus est Ad-α1AT.
